(19) Europäisches Patentamt
European Patent Office
Office européen des brevets

(11) Numéro de publication : 0 347 283 B1

# (12) FASCICULE DE BREVET EUROPEEN

(45) Date de publication du fascicule du brevet : **04.03.92 Bulletin 92/10**

(51) Int. Cl.$^5$ : **C07C 215/76,** C07C 213/02

(21) Numéro de dépôt : **89401569.2**

(22) Date de dépôt : **07.06.89**

## (54) Procédé de préparation de dichloro-2,6 amino-4 phénol.

(30) Priorité : **15.06.88 FR 8807994**

(43) Date de publication de la demande : **20.12.89 Bulletin 89/51**

(45) Mention de la délivrance du brevet : **04.03.92 Bulletin 92/10**

(84) Etats contractants désignés : **AT BE CH DE ES FR GB GR IT LI LU NL SE**

(56) Documents cités : **CHEMICAL ABSTRACTS, vol. 107, no. 25, 21 décembre 1987, page 741, résumé no. 236232f, Columbus, Ohio, US; & JP-A-62 153 262**

(73) Titulaire : **RHONE-POULENC CHIMIE**
**25, quai Paul Doumer**
**F-92408 Courbevoie Cédex (FR)**

(72) Inventeur : **Cordier, Georges**
**50 chemin des Herminières**
**F-69340 Francheville (FR)**

(74) Mandataire : **Esson, Jean-Pierre et al**
**RHONE-POULENC CHIMIE Service Brevets Chimie 25, quai Paul Doumer**
**F-92408 Courbevoie Cedex (FR)**

EP 0 347 283 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de dichloro-2,6 amino-4 phénol. Elle concerne plus particulièrement l'hydrogénation du dichloro -2,6 nitro -4 phenol selon la réaction suivante.

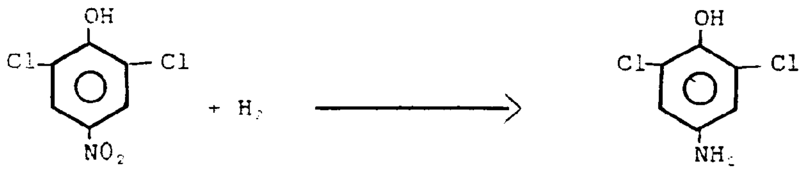

Les réactions d'hydrogénation sont des réactions connues en chimie organique mais le problème rencontré avec cette hydrogénation vient de la faible solubilité du produit final aminé.

D'autre part le dérivé nitré initial est très instable à haute température, au-dessous d'environ 150°C, il est donc nécessaire de pratiquer l'hydrogénation à basse température.

La molécule nitrée de départ a tendance à perdre facilement ses atomes de chlore par hydrodéchloration lors de l'hydrogénation.

Le produit final aminé étant un produit utilisé comme intermédiaire dans l'industrie phytosanitaire il est nécessaire d'obtenir un produit aminé contenant moins de 0,1 % de dérivé monochloré ou non chloré. Le dichloro -2,6 aminophénol présentant un point de fusion de 171°C, n'est pas purifiable par distillation ni par les moyens classiques d'extraction par solvant.

La réaction d'hydrogénation a tendance en plus des phénomènes d'hydrodéchloration à provoquer par des réactions parasites de duplication des produits secondaires fortement colorés qu'il faut à tout prix éviter car ils sont comme les produits monochlorés ou non chlorés non séparables par un procédé facile du dichloro-2,6 -2,6 aminophénol désiré.

La fragilité du produit nitré de départ, la faible solubilité du dichloro-2,6 amino-4 phénol, l'exigence de pureté du produit aminé, moins de 0,1 % de dérivés sous chlorés et moins de 100 ppm de dérivés azoïques colorés font que ce produit était inaccessible jusqu'à ce jour par des procédés faciles à mettre en oeuvre.

La présente invention a permis de trouver un solvant permettant de solubiliser les produits nitrés de départ et aminés d'arrivée et permettant de séparer par simple cristallisation le dérivé aminé sous forme pure exempte de dérivés de duplication.

La présente invention a permis aussi de choisir sélectivement le catalyseur d'hydrogénation évitant la perte de chlore par hydrodéchloration et évitant autant que le solvant la formation de dérivés azoïques.

La présente invention concerne un procédé de préparation de dichloro -2,6 amino -4 phénol qui, consiste à pratiquer une réaction d'hydrogénation du dichloro -2,6 nitro -4 phénol en présence d'un catalyseur à base d'un métal choisi parmi le palladium et le platine dans un solvant choisi parmi les éthers du diethylène glycol.

Le catalyseur à base de métal peut être un catalyseur à l'état métallique sous forme d'un oxyde, d'un sel ou sous forme d'un mélange d'entre eux.

Il peut être déposé sur un support inerte dans les conditions de la réaction. Parmi ces supports on peut citer le charbon, la silice ou l'alumine. On préfère dans les conditions de la réaction le platine déposé sur un support à base de charbon.

Parmi les solvants utilisables pour procéder à l'hydrogénation on préfère utiliser

- le monoéthers méthylique du diéthylène glycol
- le diétherméthylique du diéthylène glycol
- le monoétheréthylique du diéthylène glycol
- le diétheréthylique du diéthylène glycol.

On préfère tout particulièrement utiliser le diétherméthylique du diéthylène glycol.

On met en oeuvre, de préférence, une quantité de métal déposée sur support, inférieure à 5 % en poids et de préférence d'environ 2 % en poids.

La quantité de catalyseur exprimée en poids de métal mis en oeuvre est de préférence inférieure à 1 % en poids par rapport au dichloro-2,6 nitro-4 phénol. Pour le palladium ou le platine, on utilise plus préférentiellement, moins de 0,01 % de métal et encore plus préférentiellement moins de 0,005 % de métal.

On opère de préférence à une température comprise entre 50°C et 120°C et encore plus préférentiellement entre 80 et 100°C.

Une pression comprise entre 1 et 50 bar est avantageuse pour la mise en oeuvre de l'invention, une pression comprise entre 5 et 25 bar est préférée.

Pour une meilleure mise en oeuvre de l'invention on préfère utiliser une concentration en dichloro -2,6 nitro

-4 phénol comprise entre 250 et 1500 g/l de solvant réactionnel et de préférence d'environ 1000 g/l.

Selon un procédé de mise en oeuvre préférée, on injecte en continu le dérivé nitré dans le milieu réactionnel compte tenu de l'instabilité du dérivé nitré et du caractère très exothermique de la réaction.

Le produit désiré, le dichloro -2,6 amino -4 phénol est extrait du milieu réactionnel par addition d'eau en quantité comprise entre 100 et 200 % par rapport au volume de solvant. Par refroidissement du milieu réactionnel le dichloro -2,6 amino -4 phénol se sépare par cristallisation.

Le solvant peut être réutilisé par une nouvelle synthèse après deshydratation partielle de façon à ne laisser substituer qu'au maximum 10 % en poids d'eau.

La présente invention sera plus complètement décrite à l'aide de l'exemple suivant qui ne doit pas être considéré comme limitatif de l'invention.

EXEMPLE 1

Dans un réacteur en acier inoxydable d'une capacité de 4,0 litres équipé d'une agitation permettant d'assurer un très bon transfert de l'hydrogène gazeux dans la phase liquid, on introduit :

665 g d'ether methylique du diethylène glycol

2 g d'un catalyseur à 2,2 % de Pt deposé sur un charbon de grande surface spécifique

Le réacteur est ensuite purgé de l'air qu'il contient par un balayage convenable à l'azote, puis l'azote est à son tour éliminé par un balayge convenable à l'hydrogène.

La température est alors portée à 100°C en même temps qu'on introduit l'hydrogène pour que la pression totale à la température de 100°C soit de 10 bar.

On injecte alors à une vitesse constante et en 320 minutes une solution maintenue à 60°C de dichloro-2,6 nitro-4 phénol constitué par :

| | |
|---|---|
| . Dichloro-2,6 nitro-4 phénol | 1 747 g (2,6 moles) |
| . Diether méthylique du diethylène glycol | 913 g |

Le Dichloro-2,6 nitro-4 phénol est hydrogéné au fur et à mesure de son introduction.

On maintient les conditions de température et de pression encore 10 minutes après la fin de l'injection du composé nitré.

Le réacteur est dégazé et son contenu est filtré à l'abri de l'air et de la lumière à 80°C sous $N_2$.

Le filtrat est mélangé sous agitation et en refroidissant à 20°C avec 3360 g d'eau.

Le Dichloro-2,6 amino-4 phénol précipite. Il est filtré, essoré et lavé par 1500 g d'eau, puis séché sous 20 torrs à 60°C.

On isole aussi 1360 g (7,64 mole) soit avec un rendement de 91% un Dichloro-2,6 amino-4 phénol de spécification conforme

. titre en chloro-2 amino-4 phénol < 0,01 % (0,007 %)

. titre global par analyse > 99,5 %
(chromatographie en phase liquide)

. teneur en azote et azoxy chloré 90-95 ppm

. Couleur selon l'indice GARDNER env. 11
(à 5 % en solution dans le méthanol)

La distillation du filtrat jusqu'à obtenir un poids voisin de 460 g permet après addition d'une quantité équivalente d'eau de précipiter une nouvelle quantité (63 g) de Dichloro-2,6 amino-4 phénol qui après un lavage

à l'eau et séchage représente un rendement de 4,2 %.

Le titre de ce Dichloro-2,6 amino-4 phénol est > 99% par analyse chromatique liquide toute performance.

Il contient 0,1 % de chloro-2 amino-4 phénol et 220-230 ppm d'azote et d'azoxy chlorés.

Sa couleur exprimée selon l'indice de GARDNER est voisine de 14.

EXEMPLE 1 comparatif

Dans le même réacteur et dans les conditions de l'exemple 1, on charge :

| | |
|---|---|
| . méthanol | 1 000 $cm^3$ |
| . monochlorobenzène | 150 $cm^3$ |
| . catalyseur à 2,2 % Pt/C (le même qu'à l'exemple 1) | 1 g |

On porte la pression à 10 bar avec de l'hydrogène et la température à 100°C comme dans l'exemple 1.

On injecte en 1 h 30 mn une solution composée de :

| | |
|---|---|
| . dichloro-2,6 nitro-4 phénol | 543 g (2,6 moles) |
| . méthanol | 366 g |
| . eau | 10 g |

On maintient sous pression d'hydrogène encore 10 minutes après la fin de l'injection tandis qu'il n'y a plus d'absorption d'hydrogène.

Le réacteur est ensuite refroidi et son contenu est dilué par 1600 ml de méthanol. Il est ensuite purgé de son hydrogène et vidé de son contenu. Le catalyseur est séparé du milieu réactionnel par filtration à 30°C et lavé par 700 ml de méthanol.

Il est ensuite nécessaire d'éliminer par l'eau le chlorobenzène et la majeure partie du méthanol (env. 2/3 du mélange solvant) pour cristalliser le dichloro-2,6 amino-4 phénol.

Celui-ci est isolé sous forme d'un précipité représentant 91 % de rendement.

La pureté du Dichloro-2,6 amino-4 phénol est de 98 %.

Il contient 0,2 % de chloro amino-4 phénol et de 1,8 % de composés résultants de réaction de duplication et conférant au produit une couleur (> 18 dans l'échelle de GARDNER) incompatible avec son utilisation ultérieure.

Parmi ces sous-produits indésirables on dose environ 650 ppm de composés azo et azoxy chlorés.

Il reste en outre dans le filtrat 9 % d'un mélange de dichloro-2,6 amino-4 phénol et de divers composés de dégradation non valorisables.

CONTRE-EXEMPLE 2

On répète l'exemple (exemple 1) en remplaçant le platine sur charbon par un catalyseur Nickel de Raney dont on introduit 10 g dans le pied de solvant placé dans le réacteur.

La durée de l'injection est de 140 mn et correspond à la vitesse de transformation de ce dernier à 100°C / 10 bar.

On maintient encore 10 minutes après la fin de l'injection et on traite l'hydrogénat comme il est dit dans l'exemple 1.

On isole par cristallisation avec un rendement de 88 % le dichloro-2,6 amino-4 phénol titrant 98 %.

Parmi les sous-produits identifiés dans le dichloro-2,6 amino-4 phénol isolé on dose 0,6% de chloro-2 amino-4 phénol et 420 ppm d'azo et d'azoxy chlorés.

Ceux-ci ainsi que d'autres sous-produits non identifiés confèrent aux cristaux une couleur brune indésirée d'indice supérieur à 15 dans l'échelle de GARDNER.

Le produit isolé n'est pas conforme avec la spécification recherchée.

## Revendications

1. Procédé de préparation de dichloro -2,6 amino -4 phénol par hydrogénation du dichloro -2,6 nitro -4 phenol en présence d'un catalyseur métallique caractérisé en ce que le catalyseur est à base de platine ou de palladium et en ce que la réaction est réalisée dans un éther du diethylène-glycol.

2. Procédé selon la revendication 1 caractérisé en ce que le catalyseur est utilisé sous forme métallique, d'un oxyde ou d'un sel.

3. Procédé selon les revendications 1 ou 2 caractérisé en ce que le catalyseur est déposé sur un support choisi parmi le charbon, la silice ou l'alumine.

4. Procédé selon la revendication 1 caractérisé en ce que le solvant est choisi parmi les éthers monométhyliques et diméthyliques du diethylène glycol.

5. Procédé selon les revendications 1 à 3 caractérisé en ce qu'on met en oeuvre moins de 0,01 % en poids de catalyseur exprimé en poids de métal par rapport au dichloro 2,6 nitro 4 phénol et de préférence moins de 0,005 %.

6. Procédé selon les revendications précédentes caractérisé en ce que la température de réaction est comprise entre 50 et 120°C et de préférence entre 80 et 100°C.

7. Procédé selon la revendication 1 caractérisé en ce que la pression réactionnelle est comprise entre 5 et 25 bars.

## Patentansprüche

1. Verfahren zur Herstellung von 2,6-Dichlor-4-aminophenol durch Hydrieren von 2,6-Dichlor-4-nitrophenol in Gegenwart eines Metallkatalysators, dadurch gekennzeichnet, daß der Kata lysator auf Basis von Platin oder Palladium vorliegt und daß die Reaktion in einem Diethylenglykolether durchgeführt wird.

2. Verfahren nach Anspruch 1, dadurch gekennfzeichnet, daß der Katalysator in Form des Metalls, eines Oxids oder eines Salzes eingesetzt wird.

3. Verfahren nach den Ansprüchen 1 oder 2, dadurch gekennzeichnet, daß der Katalysator auf einem Träger aufgebracht ist, der aus Kohle, Kieselsäure oder Tonerde bzw. $Al_2O_3$ ausgewählt ist.

4. Verfahren nach Anspruch 1, dadurch gekennzeichnet, daß das Lösungsmittel aus den Monomethyl- und Dimethylethern von Diethylenglykol ausgewählt wird.

5. Verfahren nach den Ansprüchen 1 bis 3, dadurch gekennzeichnet, daß man mindestens 0,01 Gew.-% Katalysator, angegeben als Gewicht des Metalls, bezogen auf 2,6-Dichlor-4-nitrophenol, und vorzugsweise mindestens 0,005 % einsetzt.

6. Verfahren nach den vorangehenden Ansprüchen, dadurch gekennzeichnet, daß die Reaktionstemperatur im Bereich von 50 bis 120°C, vorzugsweise im Bereich von 80 bis 100°C, liegt.

7. Verfahren nach Anspruch 1, dadurch gekennzeichnet, daß der Reaktionsdruck im Bereich von 5 bis 25 bar liegt.

## Claims

1. Process for the preparation of 2,6-dichloro-4-aminophenol by the hydrogenation of 2,6-dichloro-4-nitrophenol in the presence of a metal catalyst, characterized in that the catalyst is based on platinum or palladium and in that the reaction is carried out in a diethylene glycol ether.

2. Process according to claim 1, characterized in that the catalyst is employed in the form of metal, of an oxide or of a salt.

3. Process according to claim 1 or 2, characterized in that the catalyst is deposited onto a support chosen from charcoal, silica and alumina.

4. Process according to claim 1, characterized in that the solvent is chosen from diethylene glycol monomethyl and dimethyl ethers.

5. Process according to claims 1 to 3, characterized in that less than 0.01%, and preferably less than 0.005%, by weight of catalyst is employed, expressed as the weight of metal relative to 2,6-dichloro-4-nitrophenol.

6. Process according to the preceding claims, characterized in that the reaction temperature is between 50 and 120°C and preferably between 80 and 100°C.

7. Process according to claim 1, characterized in that the reaction pressure is between 5 and 25 bars.